# FASCICULE DE BREVET EUROPEEN

(11) **EP 2 198 845 B1**
(45) Date de publication et mention de la délivrance du brevet: **23.01.2013**
(21) Numéro de dépôt: 09179891.8
(22) Date de dépôt: 18.12.2009
(51) Int. Cl.: A61K 8/34, A61K 8/41, A61K 8/49, A61Q 5/10, A61K 8/39

(54) **Composition de teinture d'oxydation des fibres keratiniques comprenant un para-aminophenol et du dipropyleneglycol**
Oxidatives Färbemittel für Keratinfasern enthaltend ein para-Aminophenol und Dipropylenglykol
Oxydative dye composition for keratin fibres comprising a para-aminophenol and dipropylene glycol

(30) Priorité: 19.12.2008 FR 0807306
(43) Date de publication de la demande: 23.06.2010
(73) Titulaire: L'Oréal, 75008 Paris (FR)
(72) Inventeur: Giafferi, Marie, 93250, Villemomble (FR); Audousset, Marie-Pascale, 92600, Asnieres (FR); Schlosser, Isabelle, 75009, Paris (FR)
(74) Mandataire: Fevrier, Murielle Françoise E.

(56) Documents cités:
- EP-A- 1 902 703
- WO-A-2008/096497
- FR-A- 2 912 904
- FR-A- 2 915 886
- JP-A- 2001 233 748
- JP-A- 2001 302 471
- JP-A- 2006 282 524
- JP-A- 2008 074 705
- US-A1- 2003 226 217

## Description

La présente demande a pour objet une composition pour la teinture d'oxydation des fibres kératiniques.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

De nombreuses tentatives ont été faites dans le domaine de la coloration capillaire afin d'améliorer les propriétés tinctoriales à l'aide, par exemple, d'adjuvants (JP 2001302471, US 20030226217). Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et les propriétés d'application de la coloration.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de coloration d'oxydation des fibres kératiniques, présentant des propriétés tinctoriales améliorées qui permettent d'atteindre l'éclaircissement souhaitée et qui soient faciles à mélanger et à appliquer.. Par propriétés tinctoriales améliorées, on entend en particulier une amélioration au niveau de la puissance/intensité et/ou de l'homogénéité de la teinture.

Ainsi, ce but est atteint par la présente invention qui a pour objet une composition pour la teinture d'oxydation des fibres kératiniques, et en particulier des fibres kératiniques humaines telles que les cheveux, destinée à être mélangée au moment de l'emploi avec un agent oxydant, comprenant,
A) une ou plusieurs bases d'oxydation choisies parmi les para-aminophénols et leurs sels d'addition avec un acide correspondants en quantité supérieur ou égale à 1,5 % en poids du poids total de la composition avant mélange avec un agent oxydant,
B) un ou plusieurs précurseurs de colorant additionnels différents de la base d'oxydation définie sous B),
C) du dipropylèneglycol en quantité supérieure ou égale à 3,5% en poids du poids total de la composition avant mélange avec un agent oxydant.

La composition selon l'invention se fabrique sans difficultés. Elle ne présente pas de cristallisations intempestives des colorants et en particulier des paraaminophénols.

La composition conforme à la présente invention se distingue après mélange avec un agent oxydant, par ses propriétés tinctoriales améliorées. En particulier, la composition de l'invention qui s'applique sans difficulté sur les fibres kératiniques conduit à des colorations qui présentent une bonne puissance et/ou intensité et/ou une bonne homogénéité de la couleur le long de la fibre entre la pointe et la racine des cheveux (appelé aussi la sélectivité de la coloration) et/ou une bonne chromaticité.

Enfin, les colorations obtenues sont tenaces, et résistent aux diverses agressions extérieures que peuvent subir les fibres kératiniques.

La présente invention a également pour objet un procédé de teinture des fibres kératiniques mettant en oeuvre la composition conforme à l'invention.

La présente invention a aussi pour objet un dispositif à plusieurs compartiments pour la mise en oeuvre de la composition de l'invention.

La présente invention a pour objet l'utilisation pour la teinture d'oxydation des fibres kératiniques de la composition conforme à l'invention.

La composition de l'invention contient un ou plusieurs para-aminophénols et/ou un ou plusieurs de leurs sels avec un acide. A titre d'exemple, des sels de para-aminophénols, on peut citer les chlorhydrates, les bromhydrates, les sulfates, les citrates, les succinates, les tartrates, les lactates, les acétates, les alkylsulfates et les alkylsulfonates.

Parmi les para-aminophénols utilisables selon l'invention, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol.

De préférence les paraaminophénols sont choisis parmi le para-aminophénol et le 4-amino 3-méthyl phénol.

Encore plus préférentiellement le paraaminophénol de l'invention est le paraaminophénol lui-même.

Selon un mode de réalisation particulier, le ou les para-aminiphénols est ou sont de préférence présents à une concentration allant de 1,5 à 10 %, encore plus préférentiellement de 1,5 à 5.%,du poids total de la composition avant mélange avec l'agent oxydant.

Le dipropylèneglycol est de préférence présent à une concentration allant de 3,5 % à 20%, encore plus préférentiellement de 3.5 à 15%, mieux de 5% à 15% du poids total de la composition avant mélange avec l'agent oxydant.

La composition selon l'invention comprend un ou plusieurs précurseurs de colorants additionnels.

Ce ou ces précurseurs de colorants additionnels sont choisis parmi les bases d'oxydation différentes des para-aminophénols et de leurs sels avec un acide, et les coupleurs.

La ou les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho aminophénols, les bases hétérocycliques ainsi que les sels d'addition de ces composés avec un acide.

Ces bases d'oxydation peuvent être en particulier cationiques.

Les para-phénylènediamines utilisables dans le cadre de l'invention peuvent notamment être choisies parmi les composés de formule (I) suivante et leurs sels d'addition avec un acide : 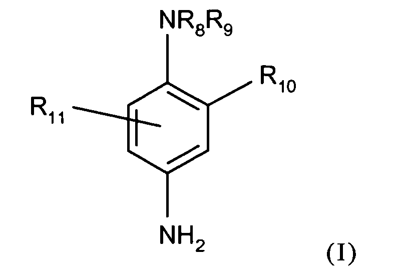 dans laquelle :
■ R8 représente un atome d'hydrogène, un radical alkyle en C1-C4, monohydroxyalkyle en C1-C4, polyhydroxyalkyle en C2-C4, alcoxy(C1-C4)alkyle(C1-C4), alkyle en C1-C4 substitué par un groupement azoté, phényle ou 4'-aminophényle ;
■ R9 représente un atome d'hydrogène, un radical alkyle en C1-C4, polyhydroxyalkyle en C2-C4, alcoxy(C1-C4)alkyle(C1-C4) ou alkyle en C1-C4 substitué par un groupement azoté ;
■ R8 et R9 peuvent également former avec l'atome d'azote qui les porte un hétérocycle azoté à 5 ou 6 chaînons éventuellement substitué par un ou plusieurs groupements alkyle, hydroxy ou uréido ;
■ R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
■ R₁₁ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les para-phénylènediamines de formule (I) ci-dessus, on peut plus particulièrement citer la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthyl-aniline, la 4-N,N-bis-(β-hydroxyéthyl)amino-2-méthyl-aniline, la N,N bis β-hydroxyéthyl paraphénylènediamine, la 4-N,N-bis-(β-hydroxyéthyl)-amino-2-chloro-aniline, la 2-β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl, β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 2-méthyl-1-N-β-hydroxyéthyl-para-phénylènediamine, et leurs sels d'addition avec un acide.

Parmi les para-phénylènediamines de formule (I) ci-dessus, on préfère tout particulièrement la para-phénylènediamine, la para-toluylènediamine, la 2-isopropyl-para-phénylènediamine, la 2-β-hydroxyéthyl-para-phénylènediamine, la 2,6-diméthyl-para-phénylène-diamine, la 2,6-diéthyl-para-phénylènediamine, la 2,3-diméthyl-para-phénylènediamine, la 2-chloro-para-phénylènediamine, la N,N bis β -hydroxyéthyl paraphénylènediamine et leurs sels d'addition avec un acide.

On utilisera tout particulièrement la para-phénylènediamine et la para-toluylènediamine, la N,N bis β-hydroxyéthyl paraphénylènediamine et leurs sels d'addition avec un acide.

Selon l'invention, on entend par bases doubles les composés comportant au moins deux noyaux aromatiques sur lesquels sont portés des groupements amino et/ou hydroxyle.

Parmi les bases doubles utilisables à titre de bases d'oxydation dans la composition conforme à l'invention, on peut notamment citer les composés répondant à la formule (II) suivante et leurs sels d'addition avec un acide : 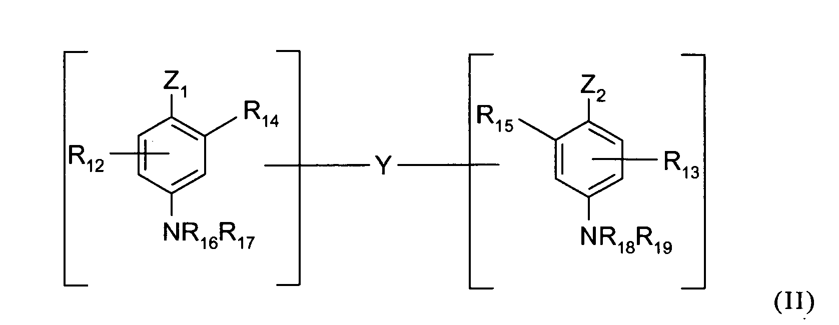 dans laquelle :
■ Z₁ et Z₂, identiques ou différents, représentent un radical hydroxyle ou -NH₂ pouvant être substitué par un radical alkyle en C₁-C₄ ou par un bras de liaison Y ;
■ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
■ R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
■ R₁₄, R₁₅, R₁₆, R₁₇, R₁₈ et R₁₉, identiques ou différents, représentent un atome d'hydrogène, un bras de liaison Y ou un radical alkyle en C₁-C₄ ;
■ étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol , le 2-(3-amino pyrazolo-[1,5-a]-pyrimidin-7-ylamino)-éthanol; le 2-(7-amino-pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol ; la 5,6-diméthyl-pyrazolo-[1,5-a]-pyrimidine-3,7-diamine ; la 2,6-diméthyl-pyrazolo-[1,5-a]-pyrimidine-3,7-diamine ; la 2,5,N7,N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme les 4,5-diaminopyrazoles tels que par exemple le 4,5-diamino-1-méthyl-pyrazole, le 4,5-diamino-1-(4'-chlorobenzyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl-pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole et le 4-amino-5-(2'-aminoéthyl)amino-1,3-diméthyl-pyrazole ; le 3,4-diamino-pyrazole ; le 4-amino-1,3-diméthyl-5-hydrazino-pyrazole ; les 3,4,5-triaminopyrazoles tels que par exemple le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole ; et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels d'addition.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels d'addition.

A titre de bases d'oxydation cationiques utilisables dans les compositions selon l'invention, on peut citer par exemple les composés suivants : les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, ainsi que les bases hétérocycliques cationiques, ces composés portant au moins un atome d'azote quaternaire.

De préférence, les bases d'oxydation cationiques utilisables dans les compositions selon l'invention sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire porteuse d'un noyau pyrrolidinique, la molécule possédant au moins un atome d'azote quaternisé. De telles bases sont, par exemple, décrites dans le document EP-A-1 348 695.

La composition selon l'invention comprend de préférence une quantité totale de bases d'oxydation allant de 0,0005 à 12 % en poids par rapport au poids total de la composition. De préférence, elle comprend une quantité totale de bases d'oxydation allant de 0,005 à 8 % en poids, et mieux encore de 0,05 à 5 % en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que, par exemple, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Selon un mode de réalisation, le précurseur de colorants est choisi parmi les bases d'oxydation para-phénylènediamines, les bases hétérocycliques, ainsi que les sels d'addition de ces composés avec un acide

La composition selon l'invention comprend généralement une quantité totale de coupleurs allant de 0,0001 à 15 % en poids par rapport au poids total de la composition. De préférence, elle comprend une quantité totale de coupleurs allant de 0,001 à 10 % en poids, et mieux encore de 0,01 à 8 % en poids, par rapport au poids total de la composition.

Les bases d'oxydation et coupleurs peuvent être présents dans les compositions de l'invention, sous forme de sels d'addition, et en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs contiennent une ou plusieurs fonctions acide carboxylique ou sulfonique, des sels d'addition avec une base sont envisageables. Les sels d'addition avec une base utilisables dans le cadre des compositions tinctoriales de l'invention sont alors notamment ceux obtenus avec de la soude, de la potasse, de l'ammoniaque ou des amines.

Selon un mode de réalisation particulier de l'invention, la composition comprend un ou plusieurs coupleurs.

La composition de l'invention peut comprendre un ou plusieurs agents alcalins. Ce ou ces agents alcalins sont par exemple choisis parmi l'ammoniaque, les carbonates ou bicarbonates alcalins, les alcanolamines telles que les mono-, di- et triéthanolamines ainsi que leurs dérivés, les éthylènediamines oxyéthylénées et/ou oxypropylénées, les hydroxydes de sodium ou de potassium, les acides aminés et en particulier les acides aminés basiques comme l'arginine ou la lysine et les composés de formule (III) suivante : 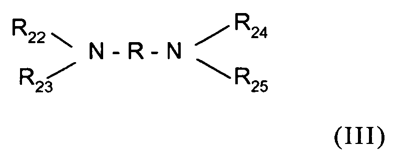 dans laquelle :
■ R est un reste propylène éventuellement substitué par un groupement hydroxyle ou un radical alkyle en C₁-C₄ ;
■ R₂₂, R₂₃, R₂₄ et R₂₅, identiques ou différents, représentent un atome d'hydrogène, un radical alkyle en C₁-C₄ ou hydroxyalkyle en C₁-C₄.

Selon un mode de réalisation particulier, la composition contient en tant qu'agents alcalins au moins une amine organique, de préférence au moins une alkanolamine. Lorsque la composition contient plusieurs agents alcalins dont une alkanolamine et de l'ammoniaque ou un de ses sels, la ou les amines organiques sont de préférence majoritaire en poids par rapport à la quantité ammoniac.

Selon un mode de réalisation particulier, la composition contient une faible quantité d'ammoniaque, voire pas d'ammoniaque. Selon ce mode de réalisation, la composition contient de préférence une ou plusieurs alcanolamines, notamment la monoéthanolamine.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques, et leurs sels d'addition. Ces colorants directs peuvent être de nature non- ionique, anionique ou cationique.

La composition peut aussi contenir d'autres composés constituant le milieu de coloration. Ce milieu de coloration outre le dipropylène glycol comprend généralement de l'eau ou un mélange d'eau et d'un ou plusieurs solvant(s) organique(s) autres que le dipropylèneglycol, de préférence hydrosolubles.

A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, l'hexylène glycol, ainsi que les alkyléthers de diéthylèneglycol comme par exemple, le monoéthyléther ou le monobutyléther du diéthylèneglycol. Les solvants peuvent alors être présents dans des concentrations comprises entre environ 0,01 à 35 % en poids et, de préférence, entre environ 0,1 et 25 % en poids par rapport au poids total de la composition.

De préférence les compositions de l'invention contiennent un ou plusieurs solvants organiques additionnels choisis parmi l'éthanol, le propylène glycol et l'hexylène glycol.

Au moment de l'emploi, la composition de l'invention est mélangée à un agent oxydant, par exemple au moyen d'une composition qui comprend un ou plusieurs agents oxydants.

Un tel agent oxydant est choisi par exemple parmi les peroxydes tels que le peroxyde d'hydrogène, le peroxyde d'urée, les bromates ou ferricyanures de métaux alcalins, les persels tels que les perborates, percarbonates et les persulfates. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40 volumes.

Selon un mode de réalisation particulier, la composition après mélange contient une quantité de dipropylèneglycol supérieure à 0,5 %.

Selon une variante, la composition après mélange avec le ou les agents oxydants contient au moins 25 % de corps gras, de préférence au moins 30 %.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins un enchaînement de 'au moins deux groupes siloxanes ou comportent une ou plusieurs chaines hydrocarbonées comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline et le décaméthylcyclopentasiloxane.

Selon cette variante, la composition comprend au moins 25 % de corps gras différents des acides gras.

Les corps gras sont notamment choisis parmi les alcanes inférieurs , les alcools gras, les esters d'acide gras, les esters d'alcool gras, les huiles non siliconées en particulier les huiles minérales, végétales, animales ou synthétiques, les cires non siliconées, les silicones.

I1 est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de 6 à 16 atomes de carbone et sont linéaires ou ramifiés, éventuellement cycliques. A titre d'exemple, les alcanes peuvent être choisis parmi l'hexane et le dodécane et les les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque
ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique ayant au moins 16 atomes de carbone, tels que les huiles de paraffine, la vaseline, l'huile de vaseline, les polydécènes, le polyisobutène hydrogéné tel que Parléam^{®} .
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

Les alcools gras utilisables dans la composition de l'invention sont saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters sont les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.
   On peut également citer les cyclocopolymères du type diméthylsiloxanes/méthylalkylsiloxane, tel que la SILICONE VOLATILE^{®} FZ 3109 commercialisée par la société UNION CARBIDE, de formule :
   avec D" : 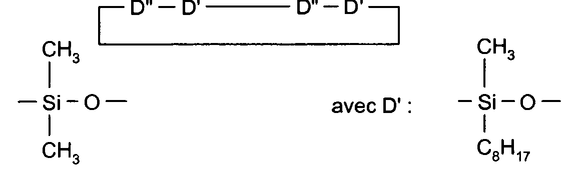 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2}
dans lesquelles R représente un alkyl possédant 1 à 16 atomes de carbone. Parmi ces produits, ceux particulièrement préférés sont ceux
dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence les corps gras sont non oxyalkylénés et non glycérolés.

Plus particulièrement, les corps gras sont choisis parmi les composés liquides ou pâteux à température ambiante et à pression atmosphérique

De préférence, le corps gras est un composé liquide à la température de 25°C et à la pression atmosphérique.

Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de la composition selon l'invention sont non siliconés.

La composition conforme à l'invention peut contenir en outre un ou plusieurs adjuvant(s) utilisé(s) classiquement dans les compositions pour la teinture des cheveux.

Par "adjuvant", on entend un additif, différent des composés précités.

A titre d'exemples d'adjuvants utilisables, on peut citer les agents tensio-actifs anioniques, cationiques, non-ioniques, amphotères, zwittérioniques ou leurs mélanges ; les polymères anioniques, cationiques, non-ioniques, amphotères, zwittérioniques, les agents épaississants minéraux ou organiques, et en particulier les épaississants associatifs polymériques anioniques, cationiques, non- ioniques et amphotères, autres que les celluloses associatives selon l'invention ; les agents antioxydants ou réducteurs ; les agents de pénétration ; les agents séquestrants ; les parfums ; les tampons ; les agents dispersants ; les agents conditionneurs tels que par exemple les silicones volatiles ou non volatiles, modifiées ou non modifiées ; les agents filmogènes ; les céramides ; les agents conservateurs ; les agents opacifiants ; et les agents anti-statique.

Les adjuvants ci-dessus sont en général présents en quantité comprise pour chacun d'eux entre 0,01 et 20 % en poids par rapport au poids de la composition tinctoriale.

Bien entendu, l'homme de l'art veillera à choisir le(s) éventuel(s) adjuvant(s) mentionné(s) ci-avant(s), de manière telle que les propriétés avantageuses attachées intrinsèquement aux compositions selon l'invention ne soient pas, ou substantiellement pas, altérées par l' adjonction (les adjonctions) envisagée(s).

Le pH de la composition conforme à l'invention est généralement compris entre 3 et 12 environ, de préférence entre 5 et 11 environ, préférentiellement 7 à 11. I1 peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on mélange la composition de l'invention avec une composition comprenant un ou plusieurs agents oxydants, et on applique le mélange sur les fibres kératiniques. La couleur peut être révélée à pH acide, neutre ou alcalin et l'agent oxydant peut être ajouté juste au moment de l'emploi ou il peut être mis en oeuvre simultanément ou séquentiellement aux autres composés de la composition de l'invention.

Après un temps de pose variant généralement de 1 à 60 minutes environ, de préférence 5 à 45 minutes environ, les fibres kératiniques sont rincées, éventuellement lavées au shampooing et rincées à nouveau, puis séchées.

Le procédé de l'invention peut être mis en oeuvre à partir de 2 ou 3 compositions, une composition comprenant le ou les para-aminophénols de l'invention, le dipropylèneglycol et le ou les précurseurs de colorants additionnels et éventuellement un ou plusieurs agents alcalins , une seconde composition comprenant le ou les agents oxydants, et éventuellement le ou les corps gras, le ou les corps gras pouvant être contenus en totalité ou partiellement dans une troisième composition.

Ainsi, l'invention a aussi pour objet un dispositif à plusieurs compartiments ou "kit" de teinture dans lequel un premier compartiment contient une composition comprenant le ou les para-aminophénols, le dipropylèneglycol, le ou les précurseurs de colorant autres que les paraaminophénols et éventuellement un ou plusieurs agents alcalins et un deuxième compartiment comprenant le ou les agents oxydants, et éventuellement un ou plusieurs corps gras.

Selon un autre mode de réalisation, le dispositif de l'invention comprend un premier compartiment qui contient une composition comprenant le ou les para-aminophénols, le dipropylèneglycol, le ou les précurseurs de colorant autres que les paraaminophénols et éventuellement un ou plusieurs agents alcalins, un deuxième compartiment comprenant le ou les agent oxydants, et un troisième compartiment contenant un ou plusieurs corps gras. Dans ce mode de réalisation, la composition comprenant le ou les corps gras peut être anhydre. On entend, par composition anhydre, au sens de l'invention, une composition cosmétique présentant une teneur en eau inférieure à 5 % en poids, de préférence inférieure à 2% en poids et de manière encore plus préférée inférieure à 1% en poids par rapport au poids de ladite composition. I1 est à noter qu'il s'agit plus particulièrement d'eau liée, comme l'eau de cristallisation des sels ou des traces d'eau absorbée par les matières premières utilisées dans la réalisation des compositions selon l'invention.

La présente invention a également pour objet l'utilisation pour la teinture d'oxydation des fibres kératiniques, et en particulier des fibres kératiniques humaines telles que les cheveux, d'une composition tinctoriale telle que définie précédemment.

Les exemples qui suivent sont destinés à illustrer l'invention sans pour autant en limiter la portée.

Dans ces exemples, toutes les quantités sont indiquées en pourcent en poids de matière active (M.A.) par rapport au poids total de la composition, sauf indication contraire.

### EXEMPLES

Les compositions suivantes ont été préparées :

### Exemple 1

| **Composition 1** | **Concentration (g%)** |
|---|---|
| DISTEARDIMONIUM HECTORITE | 3 |
| OCTYLDODECANOL | 11,5 |
| GLYCOL DISTEARATE | 8 |
| HUILE DE VASELINE | 64,5 |
| PROPYLENE CARBONATE | 1 |
| LAURETH-2 | 1 |
| POLYSOBATE 21 | 11 |

| **Composition 2** | **concentration (g%)** |
|---|---|
| ACIDE DIETHYLENE TRIAMINE PENTACETIQUE, SEL PENTASODIQUE EN SOLUTION AQUEUSE A 40% | 1 TEL QUEL |
| METABISULFITE DE SODIUM | 0,7 |
| MONOETHANOLAMINE | 14,5 |
| 1-METHYL-2,5-DIAMINO BENZENE | 1,69 |
| 1-METHYL-2-HYDROXY-4BETA-HYDROXYETHYLAMINO-BENZENE | 4,17 |
| 4-AMINO-2-HYDROXYTOLUENE | 1.39 |
| 1,3-DIHYDROXYBENZENE (RESORCINOL) | 0,88 |
| p-AMINOPHENOL | 2,43 |
| NATROSOL 250 HHR (hydroxyethylcellulose) | 1,5 |
| DIPROPYLENEGLYCOL | 10 |
| ETHANOL | 15 |
| PROPYLENEGLYCOL | 5 |
| ACIDE ASCORBIQUE | 0,25 |
| EAU | Qs 100 g |

| **Composition 3** | **Concentration (g%)** |
|---|---|
| ACIDE DETHYLENE TRIAMINE PENTACETIQUE, SEL PENTASODIQUE EN SOLUTION AQUEUSE A 40 % | 0,15 TEL QUEL |
| PEROXYDE D'HYDROGENE EN SOLUTION A 50 % (EAU OXYGENEE 200 VOL.) | 12 TEL QUEL |
| STANNATE DE SODIUM | 0,04 |
| PYROPHOSPHATE DE SODIUM | 0,03 |
| HUILE DE VASELINE | 20 |
| HEXADIMETHRINE CHL ORIDE (MA á 60% dans l'eau) | 0.25 TEL QUEL |
| POLYQUATERNIUM-6 (MA a 40% dans l'eau) | 0,5 TEL QUEL |
| GLYCERINE | 0.5 |
| ALCOOL CETYLSTEARYUQUE (C16/C18 30/70) | 8 |
| ALCOOL CETYLSTEARYUQUE OXYETHYLENE (33 OE) | 3 |
| AMIDE D'ACIDES DE COLZA OXYETHYLENE (4 OE) PRCTEGE à 92.3% dans l'eau | 1,3 TEL QUEL |
| VITAMINE E | 0.1 |
| ACIDE PHOSPHORIQUE | Qs pH 2.2 |
| EAU | QSP 100 |

### Exemple 2

| **Composition 1** | **Code MP** | **Concentration (g%)** |
|---|---|---|
| DISTEARDIMONIUM HECTORITE | 2177 | 3 |
| OCTYLDODECANOL | 1092 | 11,5 |
| GLYCOL DISTEARATE | 1122 | 8 |
| HUILE DE VASEUNE | 145 B | 64,5 |
| PROPYLENE CARBONATE | 52585 | 1 |
| LAURETH-2 | 1178 D2 | 1 |
| POLYSORBATE 21 | 53608 | 11 |

| **Composition 2** | **concentration (g%)** |
|---|---|
| ACIDE DIETHYLENE TRIAMINE PFNTACETIQKUE, SEL PENTASODIOUE EN SOLUTION AQUEUSE A 40 % | 1 TEL QUEL |
| METABISULFITE DE SODIUM | 0,7 |
| MONOETHANOLAMINE | 14,5 |
| 1-METHYL 2,5-DIAMINOBENZENE | 0,464 |
| 1-METHYL-2-HYDROXY-4-SETA-HYDROXYETHYLAMINO-BENZENE | 0,58 |
| 4-AMINO-2-HYDROXYTOLUENE | 0,087 |
| 1,3-DIHYDROXYBENZENE (RESORCINOL) | 0,435 |
| p-AMINOPHENOL | 1,56 |
| 2-METHYL-1,3-DIHYDROXYBENZENE | 1,16 |
| 2-AMINO-3-HYDROXYPIRIDINE | 0.145 |
| 6-HYDROXYINDOLE | 0.174 |
| NATROSOL 250 HHR (hydroxyethylcellulose) | 1,5 |
| DIPROPYLENEGLYCOL | 10 |
| ETHANOL | 15 |
| HEXYLENEGLYCOL | 5 |
| ACIDE ASCORBIQUE | 0,25 |
| EAU | Qs 100 g |

| **Composition 3** | **Concentration (g%)** |
|---|---|
| ACIDE DETHYLENE TRIAMINE PENTACETIQUE, SEL FENTASODIQUE EN SOLUTION AQUEUSE A 40 % | 0,15 TEL QUEL |
| PEROXYDE D'HYDROGENE EN SOLUTION A 50 % (EAU OXYGENEE 200 VOL.) | 12 TEL QUEL |
| STANNATE DE SODIUM | 0,04 |
| PYROPHOSPHATE DE SODIUM | 0,03 |
| HUILE DE VASELINE | 20 |
| HEXADIMETHRINE CHLORIDE (MA à 60% dans l'eau) | 0,25 TEL QUEL |
| POLYQUATERNIUM-6 (MA à 40% dans l'eau) | 0,5 TEL QUEL |
| GLYCERINE | 0,5 |
| ALCOOL CETYLSTEARYUQUE (C16/C18 30/70) | 8 |
| ALCOOL CETYLSTEARYUQUE OXYETHYLENE (33 OE) | 3 |
| AMIDE D'ACIDES DE COLZA OXYETHYLENE (4 OE) PROTEGE à 92,3 % dans l'eau | 1,3 |
| VITAMINE E | 0.1 |
| ACIDE PHOSPHORIQUE | Qs pH 2.2 |
| EAU | OSP 100 |

Dans chacun des exemples, les compositions 1,2 et 3 sont mélangées au moment de l'emploi dans les proportions suivantes : 10 g de la composition 1 avec 4 g de la composition 2 et 16 g de la composition 3. Le mélange est appliqué sur des mèches de cheveux gris naturels à 90 % de cheveux blancs à raison de 10 g de mélange pour 1 g de cheveux. Après 30 min de pause, les cheveux sont rincés, lavés avec un shampooing standard et séchés.

La coloration capillaire est évaluée de manière visuelle.

| | | |
|---|---|---|
| **Exemple 1** | Châtain foncé | Rouge acajou |
| **Exemple 2** | Blond | Cuivré doré |

### EXEMPLE 3

On mélange poids pour poids les compositions 2 et 3 de l'exemple 2 et on applique le mélange obtenu sur des cheveux gris à 90% de blancs naturels ou permanentés à raison de 10 g de mélange pour 1 g de cheveux

Après 30 min de pause, les cheveux sont rincés, lavés avec un shampooing standard et séchés. On obtient en final sur les cheveux une coloration peu sélective.

## Revendications

1. Composition pour la teinture d'oxydation des fibres kératiniques, et en particulier des fibres kératiniques humaines telles que les cheveux, destinée à être mélangée au moment de l'emploi avec un agent oxydant, comprenant
A) une ou plusieurs bases d'oxydation choisies parmi les para-aminophénols ou leurs sels d'addition avec un acide correspondants en quantité supérieur ou égale à 1,5 % en poids du poids total de la composition avant mélange avec un agent oxydant,
B) un ou plusieurs précurseurs de colorant additionnels différents de la base d'oxydation définie sous A),
C) du dipropylèneglycol en quantité supérieure ou égale à 3,5% en poids du poids total de la composition avant mélange avec un agent oxydant.

2. Composition selon la revendication 1 **caractérisé en ce que** le ou les paraaminophénols sont choisis parmi le para-aminophénol et le 4-amino 3-méthyl phénol et de préférence le paraaminophénol

3. Composition selon les revendication 1 ou 2 **caractérisée en ce que** le ou les para-aminophénols est ou sont présents à une concentration allant de 1,5 à 10 %, de préférencede 1,5 à 5.%,du poids total de la composition.

4. Composition selon selon l'une quelconque des revendications précédentes **caractérisée en ce que** le dipropylèneglycol est présent à une concentration allant de 3,5 % à 20%, de préférence de 3.5 à 15%, mieux de 5% à 15% du poids total de la composition.

5. Composition selon l'une quelconque des revendications précédentes, **caractérisée en ce que** le précurseur de colorant additionnel est choisi parmi les bases d'oxydation et les coupleurs.

6. Composition selon la revendication précédente, **caractérisée en ce que** le précurseur de colorant est choisi parmi les bases d'oxydation ortho- et para-phénylènediamines, les bases doubles, les ortho-aminophénols, les bases hétérocycliques, ainsi que les sels d'addition de ces composés avec un acide.

7. Composition selon la revendication précédente, **caractérisée en ce que** le précurseur de colorants est choisi parmi les bases d'oxydation para-phénylènediamine, les bases hétérocycliques, ainsi que les sels d'addition de ces composés avec un acide.

8. Composition selon la revendication 5, **caractérisée en ce que** le précurseur de colorant est choisi parmi les coupleurs méta-aminophénols, les méta-phénylènediamines, les méta-diphénols, les naphtols, les coupleurs hétérocycliques et les sels d'addition de ces composés avec un acide, de préférence un coupleur aminophénol et/ou méta-phénylènediamine.

9. Composition selon l'une quelconque des revendications précédentes comprenant un agent alcalin.

10. Composition selon la revendication 9 dans laquelle l'agent alcalin est l'ammoniac ou une alcanolamine, de préférence une alcanolamine.

11. Composition selon l'une quelconque des revendications précédentes caractérisée en ce quelle contient contiennent un ou plusieurs solvants organiques additionnels choisis parmi l'éthanol, le propylène glycol et l'hexylène glycol.

12. Procédé de teinture des fibres kératiniques, **caractérisé en ce qu'**une composition telle que définie à l'une quelconque des revendications 1 à 11 est mélangée à une composition comprenant ou plusieurs agents oxydants, puis appliquée sur les fibres kératiniques pendant une durée suffisante pour développer la coloration désirée.

13. Dispositif à plusieurs compartiments comprenant un premier compartiment contient une composition comprenant le ou les para-aminophénols, le dipropylèneglycol, le ou les précurseurs de colorant autres que les paraaminophénols et éventuellement le ou les agents alcalins, un second compartiment comprenant le ou les agents oxydants, et éventuellement un ou plusieurs corps gras ; les para-aminophénols, le dipropylèneglycol, le ou les précurseurs de colorants étant tels que définis selon l'une quelconque des revendications précédentes.

14. Utilisation pour la teinture d'oxydation des fibres kératiniques d'une composition telle que définie à l'une quelconque des revendications 1 à 11.

## Claims

1. Composition for the oxidation dyeing of keratin fibres, and in particular human keratin fibres such as the hair, intended to be mixed at the time of use with an oxidizing agent, comprising:
A) one or more oxidation bases chosen from para-aminophenols or the corresponding addition salts thereof with an acid, in an amount of greater than or equal to 1.5% by weight of the total weight of the composition before mixing with an oxidizing agent,
B) one or more additional dye precursors different from the oxidation base defined in A),
C) dipropylene glycol in an amount of greater than or equal to 3.5% by weight of the total weight of the composition before mixing with an oxidizing agent.

2. Composition according to Claim 1, **characterized in that** the para-aminophenol(s) is (are) chosen from para-aminophenol and 4-amino-3-methylphenol, and preferably para-aminophenol.

3. Composition according to Claim 1 or 2, **characterized in that** the para-aminophenol(s) is or are present at a concentration ranging from 1.5% to 10%, preferably 1.5% to 5%, of the total weight of the composition.

4. Composition according to any one of the preceding claims, **characterized in that** the dipropylene glycol is present at a concentration ranging from 3.5% to 20%, preferably from 3.5% to 15%, and better still from 5% to 15%, of the total weight of the composition.

5. Composition according to any one of the preceding claims, **characterized in that** the additional dye precursor is chosen from oxidation bases and couplers.

6. Composition according to the preceding claim, **characterized in that** the dye precursor is chosen from ortho- and para-phenylenediamine oxidation bases, double bases, ortho-aminophenols, heterocyclic bases, and also the addition salts of these compounds with an acid.

7. Composition according to the preceding claim, **characterized in that** the dye precursor is chosen from para-phenylenediamine oxidation bases, heterocyclic bases, and also the addition salts of these compounds with an acid.

8. Composition according to Claim 5, **characterized in that** the dye precursor is chosen from meta-aminophenol couplers, meta-phenylenediamines, meta-diphenols, naphthols, heterocyclic couplers and the addition salts of these compounds with an acid, preferably an aminophenol coupler and/or meta-phenylenediamine.

9. Composition according to any one of the preceding claims, comprising an alkaline agent.

10. Composition according to Claim 9, in which the alkaline agent is ammonia or an alkanolamine, preferably an alkanolamine.

11. Composition according to any one of the preceding claims, **characterized in that** it contains one or more additional organic solvents chosen from ethanol, propylene glycol and hexylene glycol.

12. Process for dyeing keratin fibres, **characterized in that** a composition as defined in any one of Claims 1 to 11 is mixed with a composition comprising one or more oxidizing agents, and then applied to the keratin fibres for a period of time sufficient to develop the desired colouring.

13. Multicompartment device comprising a first compartment containing a composition comprising the para-aminophenol(s), the dipropylene glycol, the dye precursor(s) other than para-aminophenols and, optionally, the alkaline agent(s), and a second compartment comprising the oxidizing agent(s) and, optionally, one or more fatty substances; the para-aminophenols, the dipropylene glycol and the dye precursor(s) being as defined according to any one of the preceding claims.

14. Use of a composition as defined in any one of Claims 1 to 11, for the oxidation dyeing of keratin fibres.

## Patentansprüche

1. Zusammensetzung zur Oxidationsfärbung von Keratinfasern und insbesondere von menschlichen Keratinfasern, wie Haaren, die dazu bestimmt ist, zum Zeitpunkt des Gebrauchs mit einem Oxidationsmittel gemischt zu werden und folgendes umfasst
A) eine oder mehrere Oxidationsbasen, die aus para-Aminophenolen oder deren entsprechenden Additionssalzen mit einer Säure ausgewählt werden, in einer Menge von mehr als oder gleich 1,5 Gew.-%, bezogen auf das Gesamtgewicht der Zusammensetzung vor dem Mischen mit dem Oxidationsmittel,
B) eine oder mehrere zusätzliche Farbstoff-Vorstufe(n), die sich von der Oxidationsbase unter A) unterscheiden,
C) Dipropylenglykol in einer Menge von mehr als oder gleich 3,5 Gew.-%, bezogen auf das Gesamtgewicht der Zusammensetzung vor dem Mischen mit dem Oxidationsmittel.

2. Zusammensetzung nach Anspruch 1, **dadurch gekennzeichnet, dass** das oder die para-Aminophenol(e) aus para-Aminophenol und 4-Amino-3-methylphenol und vorzugsweise aus para-Aminophenol ausgewählt wird/werden.

3. Zusammensetzung nach Anspruch 1 oder 2, **dadurch gekennzeichnet, dass** das oder die para-Aminophenol(e) in einer Konzentration von 1,5 bis 10%, vorzugsweise 1,5 bis 5%, bezogen auf das Gesamtgewicht der Zusammensetzung, vorliegt/vorliegen.

4. Zusammensetzung nach einem der vorhergehenden Ansprüche, **dadurch gekennzeichnet, dass** Dipropylenglykol in einer Konzentration von 3,5% bis 20%, vorzugsweise 3,5 bis 15%, noch besser von 5% bis 15%, bezogen auf das Gesamtgewicht der Zusammensetzung, vorliegt.

5. Zusammensetzung nach einem der vorhergehenden Ansprüche, **dadurch gekennzeichnet, dass** die zusätzliche Farbstoff-Vorstufe aus Oxidationsbasen und Kupplern ausgewählt wird.

6. Zusammensetzung nach dem vorhergehenden Anspruch, **dadurch gekennzeichnet, dass** die Farbstoff-Vorstufe aus den Oxidationsbasen ortho- und para-Phenylendiaminen, Doppelbasen, ortho-Aminophenolen, heterocyclischen Basen sowie den Additionssalzen dieser Verbindungen mit einer Säure ausgewählt wird.

7. Zusammensetzung nach dem vorhergehenden Anspruch, **dadurch gekennzeichnet, dass** die Farbstoff-Vorstufe aus den Oxidationsbasen para-Phenylendiamin, heterocyclischen Basen sowie den Additionssalzen dieser Verbindungen mit einer Säure ausgewählt wird.

8. Zusammensetzung nach Anspruch 5, **dadurch gekennzeichnet, dass** die Farbstoff-Vorstufe aus den Kupplern meta-Aminophenolen, meta-Phenylendiaminen, meta-Diphenolen, Naphtholen, heterocyclischen Kupplern und den Additionssalzen dieser Verbindungen mit einer Säure, vorzugsweise aus einem Aminophenol- und/oder meta-Phenylendiamin-Kuppler, ausgewählt wird.

9. Zusammensetzung nach einem der vorhergehenden Ansprüche, die ein alkalisches Mittel umfasst.

10. Zusammensetzung nach Anspruch 9, wobei das alkalische Mittel Ammoniak oder ein Alkanolamin, vorzugsweise ein Alkanolamin, ist.

11. Zusammensetzung nach einem der vorhergehenden Ansprüche, **dadurch gekennzeichnet, dass** sie ein oder mehrere zusätzliche organische Lösungsmittel enthält, die aus Ethanol, Propylenglykol und Hexylenglykol ausgewählt sind.

12. Verfahren zum Färben von Keratinfasern, **dadurch gekennzeichnet, dass** man eine Zusammensetzung nach einem der Ansprüche 1 bis 11 mit einer Zusammensetzung mischt, die ein oder mehrere Oxidationsmittel umfasst, und dann auf die Keratinfasern für eine Dauer aufbringt, die ausreicht, dass sich die gewünschte Farbe entwickelt.

13. Vorrichtung mit mehreren Kompartimenten, die ein erstes Kompartiment umfasst, das eine Zusammensetzung enthält, die das oder die para-Aminophenol(e), das Dipropylenglykol, die Farbstoff-Vorstufe(n) bei denen es sich nicht um para-Aminophenole handelt und gegebenenfalls das oder die alkalische(n) Mittel umfasst, ein zweites Kompartiment, das das oder die Oxidationsmittel und gegebenenfalls einen oder mehrere Fettkörper umfasst, wobei die para-Aminophenole, das Dipropylenglykol, die Farbstoff-Vorstufe(n) wie in einem der vorhergehenden Ansprüche definiert sind.

14. Verwendung einer Zusammensetzung nach einem der Ansprüche 1 bis 11 zur Oxidationsfärbung von Keratinfasern.
